# EUROPEAN PATENT SPECIFICATION

(11) **EP 2 121 645 B1**
(45) Date of publication and mention of the grant of the patent: **26.10.2011**
(21) Application number: 08707857.2
(22) Date of filing: 10.01.2008
(51) Int. Cl.: C07D 301/10, C07D 301/22, B01J 23/50, B01J 23/847, B01J 35/00, B01J 23/89

(54) **CATALYST SYSTEM AND PROCESS FOR THE PRODUCTION OF EPOXIDES**
KATALYSATORSYSTEM UND VERFAHREN ZUR HERSTELLUNG VON EPOXIDEN
SYSTÈME CATALYSEUR ET PROCÉDÉ POUR LA PRODUCTION D'ÉPOXYDES

(30) Priority: 11.01.2007 EP 07250111
(43) Date of publication of application: 25.11.2009
(73) Proprietor: CENTRE NATIONAL DE LA RECHERCHE SCIENTIFIQUE (CNRS), 75016 Paris (FR)
(72) Inventor: BASSET, Jean-Marie, F-69300 Caluire (FR); LLINAS, Jean-Richard, F-13008 Marseille (FR); MAUVEZIN, Mathias, F-13500 Martigues (FR); TAOUFIK, Mostafa, F-69100 Villeurbanne (FR); THIVOLLE-CAZAT, Jean, F-69270 Fontaine/Saone (FR)
(74) Representative: Colombet, Alain André
(86) International application number: PCT/EP2008/050235
(87) International publication number: WO 2008/084075

(56) References cited:
- EP-A- 0 605 251
- EP-A- 0 719 580
- US-A- 4 440 631
- US-A- 5 747 410
- US-A1- 2002 161 249

## Description

The present invention relates to a catalyst system which is a mixture of at least two catalytic species, and also to a process for the production of epoxides, in particular for the production of epoxides from an alkane or a mixture of an alkane and an alkene using said catalyst system.

The production of epoxides from alkenes by partial oxidation over a suitable catalyst is a well-known and widely operated commercial process.

The epoxidation of ethylene to ethylene oxide for example is believed to operate commercially exclusively using supported silver epoxidation catalysts.

An alternative desired route to epoxides would be from the corresponding alkanes. There are significant incentives to develop such a process due to the relatively low cost of alkanes compared to alkenes, but the low reactivity of alkanes to epoxidation has limited the developments in this area.

EP 0850936 A1 and US 4,990,632 are examples of two-stage processes for converting an alkane to the corresponding epoxide. In the methods described in EP 0850936 A1 and US 4,990,632 the alkane must first he dehydrogenated to the corresponding alkene in one or more dehydrogenation reaction steps, and this alkene then reacted with oxygen is a subsequent oxidation reaction step to form the corresponding epoxide. Thus, these documents describe processes with at least two reactors for performing different reactions.

US 2002/0161249 discloses a process for the conversion of alkanes into alkylene oxides, in particular the conversion of propane into propylene oxide. The catalysts are alkaline earth metal carbonate supported silver catalysts that incorporate a promoting amount of a rare-earth metal promoter, a halide promoter and an alkali metal nitrate.

It has now been found that an epoxide can be formed from an alkane by contacting an alkane or a mixture of an alkane and an alkene with a mixed catalytic bed in a reactor.

Thus, in a first aspect, the present invention provides a process for the production of an epoxide from an alkane or a mixture comprising an alkane and an alkene, which process comprises contacting said alkane or mixture comprising said alkane and said alkene and a source of oxygen with a catalyst system comprising a mixture of at least two catalytic species, the first catalytic species providing dehydrogenation activity chosen among the metal oxides (MO's) or the metal oxides mixtures and the second catalytic species providing epoxidation activity and comprising silver.

The present invention provides an improved process for the production of an epoxide which uses a catalyst system which is a mixture of at least two catalytic species to produce an epoxide from the corresponding alkane.

"Mixture of at least two catalytic species", as used herein, means that the catalytic species providing dehydrogenation activity and the catalytic species providing epoxidation activity are mixed in the catalyst system, rather than provided sequentially. In effect, the mixture combines a "conventional" epoxidation catalyst with a dehydrogenation catalyst, and reference herein to "catalysts" should therefore be understood to also equate to "catalytic species". In particular, the first catalytic species will hereinafter be referred to as a dehydrogenation catalyst and the second catalytic species will hereinafter be referred to as an epoxidation catalyst.

In one embodiment, the "mixture of at least two catalytic species" may be achieved by having an intimate mixture of the two separate catalysts, for example of the two catalysts on separate particles of suitable supports. In an alternative embodiment both the dehydrogenation and epoxidation catalysts (catalytic species) may be supported at different locations on a single support e.g. on the same particles, or on a larger substrate, such as a monolith or a foam to provide the mixed catalyst bed.

The catalyst system is preferably provided in the form of a fixed catalyst bed. The catalyst bed may be provided as a uniformly mixed catalyst bed or may be graded, for example such that more of one catalyst compared to the other is present at the front of the mixed catalyst bed than at the rear of the mixed catalyst bed. Alternatively, or additionally, further catalyst beds comprising increased relative levels of dehydrogenation or epoxidation functionality compared to the mixed catalyst bed may be present before or after the mixed catalyst bed

The process of the present invention allows use of a lower cost feed than a "pure" alkene, such as ethylene, but requires only a single reactor for both dehydrogenation and epoxidation reactions (avoiding any intermediate product treatment or separation that might be required in a two-stage process).

In particular, it is a significant advantage of the present invention that an alkane or a mixed alkane and alkene containing feed can be used, and that the overall reaction is conversion of an alkane to an epoxide rather than an alkene. Since alkane feeds are generally much cheaper than alkenes it has long been desired to convert alkanes directly to epoxides, but using mixed feeds in the process of the present invention can be more advantageous still. In particular, "pure" alkene or alkane feeds are often formed in the first place by treatment of mixed alkane/alkene streams e.g. cracker product streams, to separate alkanes and alkenes of the same number of carbon atoms from each other e.g. ethane from ethylene. This separation is costly. By using a mixed stream such a separation step may not be required at all, or, even if some separation is still required, the separation step may be operated at much lower severity.

The alkene in the mixture comprising an alkane and an alkene may also be provided, either partly or solely, by recycle of unreacted alkene from the process of the present invention. In fact, the reaction of the present invention typically produces a mixture of epoxide, alkene and unreacted alkane, which alkene and alkane can be recycled after separation of the epoxide, and mixed with a "fresh" alkane containing feed regardless of whether the "fresh" feed also comprises alkene in the first place.

The alkane used in the process of the present invention is preferably a C2 to C4 alkane, especially ethane or propane, with ethane being most preferred.

For avoidance of doubt, where a feed comprising a mixture of an alkane and an alkene is used, both the alkane and alkene in the mixture will have the same number of carbon atoms. Thus, the mixture comprising an alkane and an alkene may comprise a mixture of butane and butylene, a mixture of propane and propylene or a mixture of ethane and ethylene. Mixtures comprising propane and propylene or comprising ethane and ethylene are preferred, with a mixture comprising ethane and ethylene being most preferred (the epoxide product of the reaction then being ethylene oxide).

Preferably, the mixture of alkane and alkene comprises the alkene in molar ratio of up to 50%, preferably from 5 to 50%.

The source of oxygen may be any suitable source of oxygen which provides oxygen for reaction. Examples of suitable sources are molecular oxygen-containing gases, such as molecular oxygen itself and air, peroxides, such as hydrogen peroxide, ozone and nitrogen oxides, such as nitrogen monoxide (N₂O).

Other feed components, especially those conventionally fed to an epoxidation reaction, such as inert carrier gases and halogenated hydrocarbons may also be fed to the process if desired.

The process may be performed at any suitable pressure, but is preferably performed at an elevated pressure, especially of from 5 to 40 barg, more preferably of from 10 to 25 barg.

The process is usually performed at a temperature of at least 100°C. The temperature is usually less than 500 °C. Temperatures in the range 200°C to 350 °C are preferred.

The epoxidation catalyst comprises silver but may otherwise be any suitable epoxidation catalyst. The epoxidation catalyst may be supported on any suitable support, for example a support selected from silica, silica-alumina, alumina, any mixture of oxides containing alumina, ceria, zirconia, niobia, titania, silicon carbide, silicalites, zeolites, SAPO, CRAPO, ALPO, mesoporous materials such as MCM, SBA (mesoporous silicas) and MOF (metal-organic framework) materials. Silicas and aluminas are most preferred.

The silver may be promoted with various elements suitable for promotion of the epoxidation reaction. Typical, and preferred, examples are one or more of sodium, potassium, caesium, nickel, rhenium and chlorine, although others, including lithium, calcium, barium, rhodium and copper may be used.

Any suitable dehydrogenation catalyst may be used. Preferred dehydrogenation catalysts are metal oxides (MO's) or metal oxides mixtures. Preferably the metal oxide catalyst comprises one or more of Ni, Ti, Ta, Nb, Cr, V, Mn, Mo, Fe, Co, Cu, Ru, Rh, Pd, Pt, Ag, Cd, Os, Re, Ir, Au, Hg, Y, La, Ce, Pr, Nd, Sm, Sb, Sn, Zn, Bi, Pb, Tl, In, Te, Li, Na, K, Cs, Mg, Ca. Mixtures of metal oxides comprising at least one metal from Groups 5 to 11 of the Periodic Table are preferred.

The most preferred dehydrogenation catalyst comprises a mixture of nickel and niobium.

The dehydrogenation catalyst can be a supported or an unsupported catalyst. Where the dehydrogenation catalyst is a supported catalyst, it is preferably a supported metal oxide catalyst. Where the catalyst is supported and on a physically different support material to the epoxidation catalyst, the support used may be any suitable support, independent of that used for the epoxidation catalyst. Suitable supports may be selected from silica, silica-alumina, alumina, any mixture of oxides containing alumina, ceria, zirconia, niobia, titania, silicon carbide, silicalites, zeolites, SAPO, CRAPO, ALPO, mesoporous materials such as MCM, SBA (mesoporous silicas) and MOF (metal-organic framework) materials. Silicas and aluminas are most preferred.

For avoidance of doubt, as used herein, the term "dehydrogenation" includes oxidative dehydrogenation. Particularly preferred oxidative dehydrogenation catalysts are unsupported multi-component bulk metal oxides, such as described in WO 00/48971.

As a further advantage of the process of the present invention, the ability to produce an epoxide from a mixture comprising an alkane and an alkene also allows the present process to be advantageously integrated with a number of different alkane sources.

As one example, where the present invention uses ethane which is formed as a byproduct in the steam-cracking of naphtha, this ethane is conventionally recycled into the cracker. As well as the possibility to remove, or at least reduce, the requirement for an ethane/ethylene separation on such a stream, use of this ethane in the process of the present invention would have the additional beneficial effect of releasing furnace capacity for fresh naphtha cracking.

As a further example, the present invention may use ethane derived from natural gas. This ethane could be used with a significant quantity of methane present, with the methane acting as a carrier gas/diluent, as is conventionally used for current ethylene oxide technology. This therefore has the advantage that a feed may be used which does not require complete separation of methane.

It is also described a catalyst system which is a mixture of at least two catalytic species the first catalytic species providing dehydrogenation activity and the second catalytic species providing epoxidation activity and comprising silver. Preferably, both the dehydrogenation and epoxidation catalytic species are supported on a single support. Other preferred features of the catalyst system, such as preferred metals for the dehydrogenation catalyst, are as described for the first aspect of the present invention.

The catalysts may be produced by any suitable technique or techniques, including co-precipitation, impregnation, sublimation and grafting.

It is also described a method for preparing a catalyst system which is a mixture of at least two catalytic species the first catalytic species providing dehydrogenation activity and the second catalytic species providing epoxidation activity and comprising silver, said method comprising co-precipitation, impregnation, sublimation or grafting of the first and second catalytic species, or precursors thereof, onto a support.

One preferred technique for producing the supported catalysts is wet impregnation, typically followed by calcination. Additives, such as phosphorous containing salts, may be added to increase catalyst dispersion during impregnation. In a further preferred technique, the catalysts may also be produced by grafting of catalytic species to a support, for example as described in C. Copéret et al., Angew. Chem. Int. Ed., 2003, 42(2), 156, followed by calcination. This results directly in highly dispersed catalyst species.

### Example

### Catalyst Preparation

### 1) Preparation of Ni-Nb/alumina

A catalyst of mixed Ni-Nb-O oxides supported on alumina (theoretical metal loading: %Ni =15 and %Nb = 4,02) with an Nb/Ni atomic ratio equal to 0.176 was prepared by the evaporation method. Aqueous solutions containing the precursor salts, 1.666 g of nickel nitrate hexahydrate (>99%, Merck) and 0.323 g ammonium niobium oxalate (99.99%, Aldrich), in appropriate amounts were heated at 70 °C under continuous stirring for 1 h to ensure complete dissolution and good mixing of the starting compounds. Catalysts were prepared by conventional wet impregnation of the γ-Al₂O₃ support 2.33 g (Degussa, 100 m²/g) with aqueous solutions of nickel nitrate and ammonium niobium oxalate at 25 °C under continuous stirring for 30 min

The solvent was then removed by evaporation under reduced pressure, and the resulting solids were dried overnight at 120 °C and calcined in synthetic air at 450 °C for 5 h and oxygen at 450 °C for 30 min.

### 2) Preparation of Ag/alumina

The catalysts were prepared by conventional wet impregnation of 5 g of the Al₂O₃ support (Degussa, 100 m²/g) with aqueous solutions of silver nitrate, 1.181 g of AgNO₃ 99.995% (Aldrich) (theoretical metal loading %Ag=15). After impregnation, the solvent was removed by evaporation under reduced pressure, and the resulting solid was dried overnight at 120 °C and calcined in synthetic air at 450 °C for 5 h.

### Catalyst screening

The catalytic performance was measured by intimately mixing the Ni-Nb and Ag catalysts in a 1:1 ratio in a batch reactor. A reaction mixture comprising 50% C₂H₆, 10% O₂ and 40% N₂ (by volume) was passed over the catalyst mixture at 350°C and 10 bar pressure. After 30 minutes of reaction the products were analysed and ethylene oxide was observed. Comparative examples were performed under identical conditions using single beds of the respective Ni-Nb and Ag catalysts. No ethylene oxide was observed over the Ni-Nb catalyst. Small amounts of ethylene oxide were observed from the Ag catalyst alone, but significantly less than from the mixed catalyst.

## Claims

1. A process for the production of an epoxide from an alkane or a mixture comprising an alkane and an alkene, which process comprises contacting said alkane or mixture comprising said alkane and said alkene and a source of oxygen with a catalyst system comprising a mixture of at least two catalytic species, the first catalytic species providing dehydrogenation activity chosen among the metal oxides (MO's) or the metal oxides mixtures and the second catalytic species providing epoxidation activity and comprising silver.

2. A process according to claim 1 wherein a product stream comprising unreacted alkane, alkene and epoxide is formed, and the unreacted alkane and alkene are separated from the product stream and recycled.

3. A process according to claim 1 or claim 2 wherein the catalyst system is contacted with a mixture of alkane and alkene.

4. A process according to any one of the preceding claims wherein the mixed catalyst bed comprises an intimate mixture of first and second catalyst species supported on separate particles of suitable supports.

5. A process according to any one of claims 1 to 3 wherein the mixed catalyst bed comprises first and second catalyst species supported at different locations on a single support.

6. A process according to any of the preceding claims wherein the dehydrogenation catalyst is a supported or unsupported metal oxide catalyst.

7. A process according to any of the preceding claims wherein the metal oxide of the dehydrogenation catalyst is chosen among Ni, Ti, Ta, Nb, Cr, V, Mn, Mo, Fe, Co, Cu, Ru, Rh, Pd, Pt, Ag, Cd, Os, Re, Ir, Au, Hg, Y, La, Ce, Pr, Nd, Sm, Sb, Sn, Zn, Bi, Pb, Tl, In, Te, Li, Na, K, Cs, Mg, Ca.

8. A process according to any of the preceding claims wherein the dehydrogenation catalyst is a mixture of nickel and nobium oxides.

9. A process according to any of the preceding claims wherein the second catalyst comprises silver promoted with one or more of sodium, potassium, caesium, nickel, rhenium and chlorine.

## Patentansprüche

1. Verfahren zur Herstellung eines Epoxids aus einem Alkan oder einer Mischung enthaltend ein Alkan und ein Alken, wobei das Verfahren die Kontaktierung des besagten Alkans oder der Mischung enthaltend besagtes Alkan und besagtes Alken und eine Sauerstoffquelle mit einem Katalysatorsystem, das eine Mischung aus zumindest zwei katalytinchen Spezies enthält, umfasst, wobei die erste katalytische Spezies Dehydrogenierungsaktivität ausgewählt aus den Metalloxiden (MO's) oder den Metalloxidmischungen bereitstellt und die zweite katalytisch aktive Spezies Epoxidieaungaaktivität bereitstellt und Silber enthält.

2. Verfahren gemäß Anspruch 1, **dadurch gekennzeichnet, dass** ein Produktstrom enthaltend nicht umgesetztes Alkan, Alken und Epoxid gebildet wird und das nicht umgesetzte Alkan und Alken von dem Produktstrom separiert und rezykliert wird.

3. Verfahren gemäß einem der Ansprüche 1 oder 2, **dadurch gekennzeichnet, dass** das Katalysatorsystem mit einer Mischung aus Alkan und Alken kontaktiert wird.

4. Verfahren gemäß einem der vorhergehenden Ansprüche, **dadurch gekennzeichnet, dass** das gemischte Katalysatorbett ein inniges Gemisch einer ersten und zweiten Katalysatorspezies enthält, die auf separaten Partikeln geeigneter Träger getragen wird.

5. Verfahren gemäß einem der Ansprüche 1 bis 3, **dadurch gekennzeichnet, dass** das gemischte Katalysatorbett eine erste und zweite Katalysatorspezies enthält, die an verschiedenen Orten eines einzelnen Trägers getragen wird.

6. Verfahren gemäß einem der vorhergehenden Ansprüche, **dadurch gekennzeichnet, dass** der Dehydrogenierungskatalysator ein getragener oder ungetragener Metalloxidkatalysator ist.

7. Verfahren gemäß einem der vorhergehenden Ansprüche, **dadurch gekennzeichnet, dass** das Metalloxid des Dehydrogenierungskatalysators ausgewählt ist aus Ni, Ti, Ta, Nb, Cr, V, Mn, Mo, Fe, Co, Cu, Ru, Rh, Pd, Pt, Ag, Cd, Os, Re, Ir, Au, Hg, Y, La, Ce, Pr, Nd, Sm, Sb, Sn, Zn, Bi, Pb, Tl, In, Te, Li, Na, K, Cs, Mg, Ca.

8. Verfahren gemäß einem der vorhergehenden Ansprüche, **dadurch gekennzeichnet, dass** der Dehydrogenierungskatalysator eine Mischung aus Nickel- und Nioboxid ist.

9. Verfahren gemäß einem der vorhergehenden Ansprüche, **dadurch gekennzeichnet, dass** der zweite Katalysator Silber gefördert mit einem oder mehreren aus Natrium, Kalium, Caesium, Nickel, Rhenium und Chlor enthält.

## Revendications

1. Procédé de fabrication d'un époxyde à partir d'un alcane ou d'un mélange comprenant un alcane et un alcène, lequel procédé consiste à mettre en contact ledit alcane ou le mélange comprenant ledit alcane et ledit alcène et une source d'oxygène avec un système catalytique comprenant un mélange d'au moins deux espèces catalytiques, la première espèce catalytique permettant l'activité de déshydrogénation choisie parmi les oxydes métalliques (MO) ou les mélanges d'oxydes métalliques et la seconde espèce catalytique permettant l'activité d'époxydation et comportant de l'argent.

2. Procédé selon la revendication 1, dans lequel est formé un flux de produit comprenant l'alcane et l'alcène n'ayant pas réagi et l'époxyde produit, l'alcane et l'alcène n'ayant pas réagi sont séparés du flux de produit et recyclés.

3. Procédé selon la revendication 1 ou la revendication 2 dans lequel le système catalytique est mis en contact avec un mélange d'alcane et d'alcène.

4. Procédé selon l'une quelconque des revendications précédentes, dans lequel le lit de catalyseur mixte comprend un mélange intime de la première et de la seconde espèces de catalyseur supportées sur des particules séparées de supports appropriés.

5. Procédé selon l'une quelconque des revendications 1 à 3, dans lequel le lit de catalyseur mixte comprend les première et seconde espèces de catalyseur supportées à différents emplacements sur un seul support.

6. Procédé selon l'une des revendications précédentes, dans lequel le catalyseur de déshydrogénation est un catalyseur d'oxyde métallique supporté ou non supporté,

7. Procédé selon l'une quelconque des revendications précédentes dans lequel l'oxyde métallique du catalyseur de déshydrogénation est choisi parmi Ni, Ti, Ta, Nb, Cr, V, Mn, Mo, Fe, Co, Cu, Ru, Rh, Pd, Pt, Ag, Cd, Os, Re, Ir, Au, Hg, Y, La, Ce, Pr, Nd, Sm, Sb, Sn, Zn, Bi, Pb, Tl, In, Te, Li, Na, K, Cs, Mg, Ca.

8. Procédé selon l'une quelconque des revendications précédentes, dans lequel le catalyseur de déshydrogénation est un mélange d'oxydes de nickel et de nobium.

9. Procédé selon l'une quelconque des revendications précédentes, dans lequel le second catalyseur comprend de l'argent activé avec un ou plusieurs parmi le sodium, le potassium, le césium, le nickel, le rhénium et le chlore.
